# EUROPEAN PATENT SPECIFICATION

(11) **EP 2 771 045 B1**
(45) Date of publication and mention of the grant of the patent: **28.07.2021**
(21) Application number: 12844760.4
(22) Date of filing: 30.10.2012
(51) Int. Cl.: A61M 1/34, A61M 1/38, B01D 63/02

(54) **METHOD FOR THE REDUCTION OR ELIMINATION OF ONE OR MORE COMPONENTS FROM A BLOOD PRODUCT**
VERFAHREN ZUR VERRINGERUNG ODER ELIMINIERUNG EINER ODER MEHRERER BESTANDTEILE AUS EINEM BLUTPRODUKT
PROCÉDÉ DE RÉDUCTION OU D'ÉLIMINATION D'UN OU PLUSIEURS COMPOSANTS D'UN PRODUIT SANGUIN

(30) Priority: 30.10.2011 SE 1100815
(43) Date of publication of application: 03.09.2014
(73) Proprietor: Glycorex AB, 223 70 Lund (SE)
(72) Inventor: NILSSON, Kurt, S-226 53 Lund (SE)
(74) Representative: AWA Sweden AB
(86) International application number: PCT/SE2012/051178
(87) International publication number: WO 2013/066251

(56) References cited:
- EP-A1- 2 087 916
- WO-A1-95/04559
- WO-A1-95/04560
- WO-A1-95/04560
- WO-A1-97/48483
- WO-A1-2011/009555
- WO-A1-2012/142180
- WO-A2-95/03084
- DE-A1- 2 828 549
- GB-A- 2 124 511
- JP-A- 2000 167 044
- JP-A- 2010 235 496
- US-A- 5 286 449
- US-A- 6 022 477
- US-A1- 2008 185 322
- US-A1- 2010 100 027
- US-A1- 2011 094 962
- None

## Description

### Technical Field of the Invention

The present invention refers to a method for the reduction or elimination of one or more components from a blood product.

### Background Art

Different kinds of blood therapies or apheresis procedures are performed on a daily basis on a large number of patients.

The aim of the apheresis procedures is for example to reduce the level of certain blood components, such as antibodies, proteins, toxins or other components in the patient's blood, which contribute to different disease conditions, or to derive blood plasma, blood components and molecules from blood donors.

The filters used in dialysis, plasma exchange (the latter is usually shortened TPE, i.e. therapeutic plasma exchange, referred to as TPE below) and double filtration treatments (generally shortened DFPP, referred to as DFPP below), have different pore sizes, respectively.

These methods enable a separation based on the molecular weight and size of the different blood components. The methods, however, do not achieve any biospecific separation, i.e. the separation is not based on the biological specificity of each blood component.

A more specific methodology called immunoadsorption, normally shortened IA and referred to as IA below, was therefore introduced. In IA, a bio specific column is used in an extracorporeal blood treatment. A more effective and gentle treatment can thus be achieved for the patient. The column contains a beaded carrier material, referred to here and below as a matrix, to which a ligand, referred to as ligand below, which often consists of or contains at least one protein, a peptide, an antibody, or a carbohydrate structure, is covalently bound. The ligand is capable of binding the desired component for reduction, more or less specifically.

IA enables a specific treatment, i.e. a specific elimination or reduction of the component or target substance of the treatment, e.g. a reduction of immunoglobulin or other proteins in the blood.

Examples are protein A containing columns. These columns contain protein A bound to a polymeric carrier material, i.e. cross-linked agarose. Another example is immunoglobulin based columns for the specific elimination of immunoglobulins or for separation of other proteins or components from the blood plasma. Other examples are columns with covalently bound blood group saccharides with a view to specifically binding the proportion of antibodies in the blood which are specific for blood group determinants A and B, respectively, whilst other antibodies and blood components pass through the column.

During IA a plasma filter is often used (as for plasma exchange) or a centrifuge, which continuouosly separates the plasma from the blood cells, and the plasma is lead through a tube to a column where the target protein or substance bind and thus is specifically separated from other plasma components. The blood plasma that has passed through the column is continuously returned to the patient.

WO 95/04560 discloses treatment of tumors via extracorporeal blood conditioning, wherein the blood is passed through a filter.

WO 95/04559 discloses an arrangement with a membrane filter for removing substances in connection with blood purification processes.

WO 95/03084 discloses an extracorporeal affinity adsorption device for treatment of different disease conditions.

DE 2828549 discloses an apparatus for blood treatment comprising a membrane device having two semipermeable membranes.

### Summary of the Invention

The invention is defined by the features of independent method claim 1.

The present invention refers to a method for the reduction or elimination of one or more components from a blood product, wherein it comprises continuous addition of the blood product to a filter containing an inlet for the blood product, an outlet for blood product in which said one or more components have been reduced or eliminated, hollow fiber membranes having pores through which said one or more components and the blood plasma may pass outwardly, a space between the hollow fiber membranes, a blood plasma outlet, a blood plasma inlet, and a loop connected between said blood plasma outlet and said blood plasma inlet, wherein said space between the hollow fibers and the loop contains an adsorbent which specifically binds said one or more components, wherein the pores of the hollow fibers do not allow passage of the adsorbent into the hollow fiber membranes, wherein the blood plasma is continuously recirculated via the loop back to the space between the hollow fibers, and wherein when the concentration of said one or more components in the blood product which exits the filter via the blood product outlet reaches a pre-determined value, the addition of blood product is stopped.

### Brief Description of the Drawings

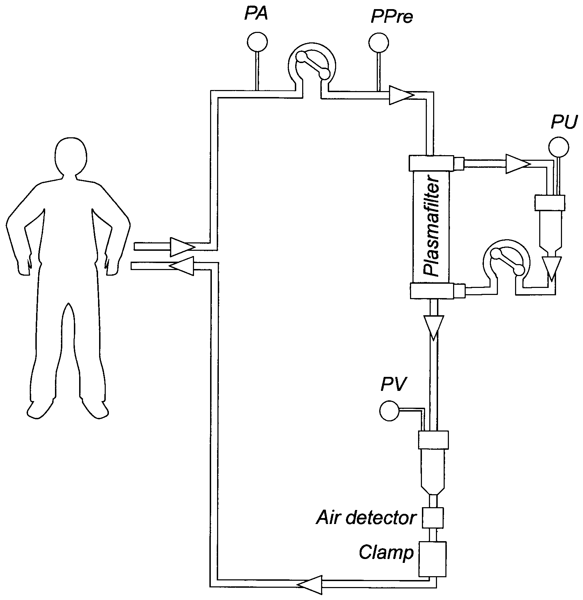

Fig 1 schematically shows one embodiment of the system used in the method according to the present invention.

### Detailed Description of Different Embodiments

In connection with the invention the adsorbent is used in a continuous process in conjunction with at least one plasma filter, for the reduction of blood components in donor blood, blood plasma, or for purification of partly purified components, such as antibodies, or proteins or for purification of any other fluid. The adsorbent binds the intended component in the blood, the blood plasma, the partly purified blood plasma, or the partly purified blood components, for example an antibody, a protein or component(s) in any other fluid.

In an optional last step the bound component is eluted from the adsorbent by for example changing the pH in the adsorbent hence breaking the specific binding between the bound component and the adsorbent. The invention also refers to the products produced in this way.

In this way the adsorbent may optionally be reused following separation from the bound product and the blood, blood plasma or the completely or partially purified blood plasma. For example, such components as antibodies or other proteins, which have been bound the adsorbent, can often be eluted by reducing the pH (by using for example 0.1 M glycine buffer having a pH of 3 or of a lower pH). The choice of elution buffer and the other conditions for elution may be made by a person skilled in the art.

As mentioned above, the invention involves the use of the adsorbent in conjunction with at least one blood or plasma filter. There is a large selection of commercially available blood or plasma filters on the market and these can optionally be used according to the invention. The choice of the filter is made depending on the actual application. For example, capacity, filter size, and flow rate range (mL per minute) of blood and blood plasma, are chosen from from the volume to be treated) and the desired treatment effect. Similarly, the size of the filter, the pore size/porosity of the filter membrane, the flow rate capacity, the pressure, the filtration capacity, and other mechanical, chemical, biochemical, medical and flow related properties, is chosen by the specialist. Other equipment used for the treatment, such as the machine (normally a so called plasma separator for use with plasma filters), is chosen by an expert. The plasma filter used for the treatment is normally connected to the machine via the normal set up used for plasma filter treatment. The machine and the tubings for the connections may also be chosen by the specialist.

Plasma filters with different capacities and pore sizes are commercially available. Non-limiting examples are plasma filters with membranes comprised of e.g. polysulfone or any other plastic.

These filters often have one blood line and one blood plasma line, i.e. an inlet and an outlet for blood (below referred to the inlet and outlet of blood respectively) and an inlet and an outlet for blood plasma (below referred to the inlet and outlet of blood plasma, respectively).

Blood filters are often cylindrically constructed with several so called hollow fibers, which run axially inside a cylindric container between the blood inlet and outlet of the filter. Whole blood or blood plasma will pass through the porous hollow fiber membranes of the filter during use. The flow rate of blood or blood plasma is controlled by separate pumps on the plasma separator. Blood plasma thus passes through the pores in the hollow fiber membranes to the space in between the hollow fibers and out through the blood plasma outlet. The hollow fibers can be made of for example a polysulfone membrane, which has pores that allow passage of plasma and plasma components. For example, blood (plasma) filters have hollow fibers with pores of a size that allow passage of IgG and IgM, (e.g. 0.1 - 0.5 µm pores or a range within this range). Even filters with smaller pores are common for the allowance of selective passage based on the size of these plasma components to be separated. The pores will not, however, allow passage of larger components such as red and white blood cells (or for example antibodies, as is the case of smaller filters which have hollow fibers of smaller pore sizes).

The adsorbent is present in the blood plasma filter between the blood inlet and outlet (i.e. in the space between the hollow fibers) as well as in a 'loop' in the form of a plastic tubing line, chosen by the specialist. The loop is connected in both of its ends to the outlet for of blood plasma and to the inlet for blood plasma, respectively, of the plasma filter. The mixture of adsorbent and the blood plasma is continuously circulated, from the loop into the blood filter (i.e. to the space between the hollow fiber membranes) and out from the blood plasma filter via the blood plasma outlet back to the loop, throughout the method performed according to the present invention. The flow rate of the mixture of blood plasma and adsorbent is controlled by a pump (as in regular plasma exchange), and also, for example, the pressure is monitored. The plasma flow rate is in one embodiment of the invention set at a higher flow rate than the flow rate of the blood line.

An example of reduction or separation of component from blood according to the invention is the following: Whole blood is continuously pumped through a blood plasma filter. A proportion of the plasma continuously passes from the whole blood inside of the hollow fibers through the pores to the space between the hollow fibers (plasma side) with the adsorbent. The components or the component one wishes to reduce in the blood or in the blood plasma comes in contact with the adsorbent and bind to the ligand in the adsorbent (which exists between the hollow fibers and in the above mentioned 'loop'). The treated plasma continuously returns to the space containing the whole blood inside the hollow fibers. The adsorbent together with the bound component, does however not pass through the pores but remains in the space between the hollow fibers and in the 'loop'.

The present invention will be explained more in detail below with reference to Fig. 1.

The circulation line connected to the filter illustrates the loop, which contains the adsorbent to which the components binds, and in this embodiment, patient plasma. The line between the patient and the plasma filter illustrates, in this embodiment, blood from the patient to the plasma filter, and the line between the plasma filter and the patient contains treated whole blood from the plasma filter back to the patient. Pumps, air detectors and pressure monitors (PA, PPre, PU, and PV) and flow directions are also illustrated in Fig. 1.

The patient shown in Fig. 1 is in an embodiment according to the present invention e.g. a blood bag containing blood donor blood or blood donor plasma, or a container with a liquid solution with a component which is desired to reduce and/or purified from the liquid solution. The plasma or the liquid can be used after reduction of the component, and alternatively, the component can be used after elution from the adsorbent. In the latter example, the component can be eluted from the adsorbent, for example by changing the pH. Proteins, such as coagulation factors, for example factor VIII, or any other protein of for example therapeutic interest, such as an antibody, can often be eluted with, for example, glycine buffer, for example 0.1 M, pH 2.2, or with any other buffer, concentration or pH chosen by the expert for the specific component.

The method according to the present invention, can in one embodiment be used with a view to producing whole blood, blood plasma, blood platelets, an intravenous immunoglobulin preparation with reduced content of, for example, blood group specific antibodies when the adsorbent contains a ligand being a blood group determinant A and/or B. In another embodiment, when the component is an antibody or any other protein, the component can be eluted from the adsorbent as exemplified above, and be used, for example, in therapeutic or analytic applications.

In addition, according to one embodiment, one or more components can be eliminated or reduced by use of the inventive method in the same way for a blood product in the form of blood plasma, a completely or partly purified blood plasma, or any aqueous liquid containing components of interest to eliminate or reduce from the blood product or to separate from the blood product. Here, the blood plasma, partially purified blood plasma or liquid is passed through the whole blood line of the blood/plasma filter and a proportion of blood plasma, partially purified blood plasma or liquid the containing component will pass through the pores into the space between the hollow membranes, and the component will bind to the adsorbent present in said space and in the loop.

The applied flow rates are determined depending on for example the plasma filter used and the current application, for example, the desired treatment time and treatment effect, and the blood, blood plasma or liquid volume to be treated.

The level of the component or components wished to be reduced will therefore be lower at the blood outlet of whole blood of the filter, compared to the concentration of component(s) at the blood inlet. Thus, the level of the component in a container with the component, will be increasingly reduced during the treatment, which is stopped when the desired treatment effect has been achieved as determined by the expert in the field.

As stated above, according to one embodiment of the present invention, one or more components can be separated or reduced during the treatment method in the same way, but starting from blood plasma, from completely or partly purified blood plasma or from an aqueous liquid. Here, the blood plasma, partially purified blood plasma or liquid is passed through the whole blood line of the blood (plasma) filter and a proportion of blood plasma, partially purified blood plasma or liquid containing the component will pass through the pores, wherein the component will bind to the adsorbent present in the space between the hollow fiber membranes and on the loop.

The applied flow rates are determined by, for example, the plasma filter used and by the current application, for example the desired treatment time and treatment effect, and the blood, blood plasma or liquid volume to be treated. This is determined by the specialist. For example, a blood flow rate (the flow rate through the blood line of the blood (plasma) filter) of 25, 50, 100 or 200 ml per minute can be chosen, or a value in between these values. Likewise, the plasma flow rate (the flow of plasma with the adsorbent in the loop and in the space between the hollow fiber membranes, is chosen by the specialist depending on the application. For example, a plasma flow of 50, 100, 200, 300 or 400 ml per minute, or a value in between these values, may be chosen. Examples of parameters the specialist will take into account are the blood volume, the size of the filter, the amount of adsorbent, the loop volume (of plasma and adsorbent) and the treatment time.

In one embodiment of the invention, the flow rate of the mixture of adsorbent and blood plasma is set higher than the blood flow rate, and a ratio of 2 or higher have, for example, been shown to give a desired treatment, for example, in the reduction of the amount of a protein or antibody.

The adsorbent containing the matrix and the ligand bound thereto can be soluble or not soluble, as mentioned above. Examples of the matrix are given below. If a soluble matrix is used, this can be, for example, dextran or be comprised of polyacrylamide with a molar mass which does not allow passage through the pores into the hollow fibers with a view to preventing passage of the adsorbent through the pores into the whole blood line. Examples of molar masses of the matrix are 2, 3, 4, 10, 20 million Daltons or higher, or a value in between these values, during the use of filters with hollow fibers with pores that do not allow passage of components of at least the mentioned molar mass. With a non-soluble matrix, for example agarose or cross-linked agarose may be used. In one embodiment beaded cross-linked agarose is used. The size of the agarose beads is chosen to be larger than the pores of the filter. Examples of matrices are given below.

The ligand can for example be one or more of the saccharides mentioned below, i.e. a peptide, a protein, an antibody, or any other ligand, which can bind to the component or components to be reduced in the blood, the blood plasma or any other fluid.

By the continuous passage through the above described system, a blood filter that contains the adsorbent described above, of for example whole blood (or through the passage through above described system of blood, blood plasma or with a completely or partly purified blood plasma or other fluid from a container), and through the continuous return of blood that has passed the blood filter, or by continuously returning to the container or continuously passing to a separate container respectively, the component or components desired to be reduced can be reduced in the plasma containing container, or a lower concentration of the component can be achieved in another type of liquid than blood or plasma, following the passage through the filter with the adsorbent.

In one embodiment, the production of the adsorbent with a covalently bound ligand is completely or partly performed according to GMP in clean rooms, and sterilization is made by autoclaving (e.g. steam sterilization). For the treatment, the adsorbent can first have been sterilized in the 'loop' or in a part of the 'loop' and thereafter been connected to the filter before the start of the treatment, or be sterilized in a container (a plastic bag with a plastic tube connection to the 'loop' or in a bottle or in a syringe for injection into the 'loop') before the treatment and thereafter be injected into, for example, the 'loop' before the treatment. The expert in the field will also determine the parameters for sterilization to achieve a validated sterilization, and this does not limit the scope of the invention. Likewise, sterile blood - or blood plasma filters, plastic linings and connectors required for the treatment and selected by the expert, will be used in one embodiment.

During the purification of blood or blood plasma or completely or partly purified blood plasma, the blood plasma filter, e.g. has a pore size that allows the passage of IgG, IgA and IgM, for example a pore size in the range 0.1 to 0.3 µm.

In conjunction with an adsorbent containing a matrix with covalently bound ligands which contain one or more of blood group A and/or blood group B saccharides, as described below, this can be used for a reduction of anti-A and or anti-B antibodies of the IgG, IgA and IgM type. The same type of filter can be used for removal of other proteins or antibodies.

The adsorbent, i.e. the adsorbent, consists of or comprises at least one matrix with at least one covalently bound ligand. Examples of ligands are ligands which contain carbohydrate structures which are able to selectively bind certain antibodies, toxins or other proteins. Examples of ligands are ligands which contain one or more of blood group A or blood group B saccharides, which are described below. Adsorbents containing these ligands can be used according to the invention for reduction of antibodies specific for blood group determinants, i.e. so called anti-A or anti-B antibodies of IgG, IgM and IgA type. Other examples are ligands which contain other carbohydrate structures, such as those found in, for example, gangliosides, other glycolipids, and glycoproteins. There are several such structures known to be able to bind to antibodies, proteins, and toxins. Examples are anti-GM1 antibodies, anti-GD1 a antibodies, antibodies specific for other sialylated carbohydrate structures, antibodies specific for other carbohydrate structures, proteins and toxins binding to for example Galα1-3Gal structures. An adsorbent with ligand containing, for example, the disaccharide structure Galα1-4Gal, can be used in connection with the invention, for example for reduction in blood or blood plasma of toxins which are formed by certain pathogenic bacteria (for example in a condition called HUS).

Other examples of ligands are proteins, such as protein A or peptide fragments thereof, with the ability to bind immunoglobulins, antibodies specific for binding of immunoglobulins or for binding of other proteins or toxins.

Some patients have developed HLA specific antibodies, i.e. antibodies towards HLA antigens. These patients are often called 'sensitized' patients, and it is known to be more difficult to perform a successful transplant to these patients. There are several HLA antigens which are characterized by specific peptides. These peptides can be used as ligands in an adsorbent and be used for reduction of HLA specific antibodies in sensitized patients, thereby facilitating transplants to these patients.

Treatment protocols are developed by the specialist in the field.

In the same way, other components can be reduced (or purified) (with the aid of other adsorbents, as exemplified above) from blood.

Treatment of different medical conditions during for example transplantation (anti-A/B/H antibody reduction, anti-HLA antibody reduction), autoimmune conditions (reduction of for example GM1 specific antibodies and other examples given below), treatment of bacterial infections (reductions of toxins exemplified below), but also for the development of, for example, antibodies and other proteins is disclosed here.

The treated volume, amount of adsorbent, flow rates, temperature, contact time, pressure, size of blood plasma filter, type of blood plasma filter will be adapted to the current application as chosen by the specialist.

The above mentioned adsorbents can also be used for binding of anti-A and or anti-B and or anti-H antibodies from human blood immunoglobulin fractions, in either purification stage, initial, intermediate or finished product (intravenous immunoglobulin). The specific volume of the adsorbent and the chosen variant of the adsorbent as described above, is chosen by the expert in the field. This depends on, for example, the desired exact application and the quantity of antibodies to be removed. The adsorbent can be used in large scale application for production of e.g. human plasma or intravenous immunoglobulins with reduced or minimal content of mentioned blood group specific antibodies.

As a non-limiting example may be mentioned the reduction of anti-A and or anti-B antibody titer in a patient's blood in connection with blood group incompatible transplantation. Depending on for example the start anti-A and or anti-B titer, and the plasma volume of the patient, the filter (i.e. normally for an adult a plasma filter for treatment of an adult) and the quantity of adsorbent with ligand containing blood group A and or blood group B determinant(s) is chosen by the expert. For example, 10, 20 or 30 mL or a quantity between these values, of for example settled, beaded crosslinked agarose containing A- and or B-ligand can be chosen for the treatment. The flow rate of the blood line can be chosen to be for example 50, 100 or 150 mL per minute or a value in between these values. The flow rate of the plasma line (loop with suspension of the agarose beads in plasma) can, for example, be 100 mL, 200, 300 or 400 mL per minute or a value in between these values depending on for example the size of the patient, the plasma filter and the chosen flow rate of the blood line. The total volume of the suspension of agarose beads in plasma is also chosen by the expert and can be chosen to be for example 100, 200 or 300 mL or a higher value or a value between these values. The plasma filtration machine is used for example to control the flow rates, the pressures of the blood and plasma lines and also for example controlling that a minimum of air enters the treatment system. The duration of treatment is determined by the desired treatment effect, in this example titer decrease) and also by for example the blood volume. A higher blood volume will normally require a longer treatment time to obtain a set treatment effect. These factors are also determined by the expert. Normally, the treatment is performed during 30 min, 1 h, 2 h, 3 h, 6h or a value between these values. Similarly, other antibodies, for example anti-HLA, anti-GM1, other anti-ganglioside antibodies, other anti-carbohydrate antibodies, other proteins or toxins, can be reduced according to the inventive method with other adsorbents, as exemplified above.

For larger plasma volumes the efficiency of the treatment can be increased by using two or more plasma filters in parallel (for two filters the blood line is divided in two lines before the filters and after the blood outlet are connected to one line), and each filter having one loop with adsorbent.

In another embodiment of the invention two filters are used in sequence in a treatment of whole blood, whole blood continously enters this filter which continously separates out plasma, the plasma line is connected to a loop (which in turn is connnected to the second filter) containing an adsorbent, as described above, the loop flow goes to the plasma inlet of the second filter, the plasma enters the space inside the hollow fibers of the second filter, and the plasma (with reduced content of the component) is continuosly returned to the whole blood outlet line from the first filter. In this case the plasma inlet of the first filter is closed and the blood line inlet of the second filter is also closed.

The quantity of ligand in the adsorbent is typically chosen to contain 0.1, 0.5, 1.0, 2.0, 10, 20 or 50 mg ligand per g dry weight of soluble adsorbent or per mL settled, beaded volume of non-soluble adsorbent, or any value between these values. The value is chosen by the expert in the field. In the examples where low molecular weight ligands (for example containing mono-, di- or oligomeric saccharide(s), or amino acid or peptide) are used covalently bound to cross-linked beaded agarose and used for reduction of proteins, such as antibodies, a value in the lower range can be chosen, for example a value of 0.1, 1, 2 or 10 mg or a value in between these values, per mL of beaded crosslinked agarose.

One embodiment of the invention is characterized by that at least the final stages of the production of the adsorbent is performed in clean rooms, and preferably the reagents and the clean room(s) used are certified according to international standards and/or requirements for the product application.

In another embodiment of the invention, the to the adsorbent bound components, such as for example anti-A and or anti-B and or anti-B antibodies, can be eluted from the adsorbent, analyzed and used for various applications, such as treatment of infections, purification or for analytical purposes.

In another embodiment, blood products obtained after treatment with the adsorbent used in the method according to the invention have a higher purity. Examples are human blood, blood plasma, and IVIG preparations, which after treatment with the adsorbent used in the method according to the invention have a reduced content of undesired component(s). As an example can be mentioned removal of anti-A and anti-B antibodies from human blood, human plasma or from IVIG preparations (or during preparation of IVIG) using as adsorbent, for example cross-linked agarose beads with covalently bound blood group A- and or blood group B containing ligands. These purified products can be used for blood transfusion, plasma transfusion, and for other treatments, e.g. treatment of immunodeficiency and certain neurological diseases.

In transplantation of blood group A1 donor in transplantation of blood group A1 donor organs or cells to A2 recipients, there is a risk that the A2 recipient contains antibodies specific towards certain variants of blood group A structures. By treating the A2 recipient with the adsorbent containing the blood group A variant, these antibodies can be reduced or eliminated thereby reducing the risk of side effects due to those antibodies after transplantation. More broadly, certain blood group O and certain blood group B patients contain antibodies not only to A-trisaccharides, but also towards longer A-structures, and these patients can be treated with an adsorbent in which the matrix contains a mixture of for example A-trisaccharide containing ligand with at least one A-tetrasaccharide-containing ligand (for example of subtype 1, 2, 3, and or 4 mentioned below) with a view to removing said antibodies and facilitate blood group incompatible transplantation from A donors. In one embodiment of the invention, this can be performed especially when there is a relatively low reduction of the titer with conventional A trisaccharide-ligand or there is a persistent rebound of antibodies.

In addition, in blood group incompatible stem cell transplantation, the product can be applied to treat blood obtained from patients with high anti-A, and or anti-B and or anti-H titers with a view to avoiding problems with anti-A, anti-B and anti-H specific antibodies.

As an example, an adsorbent may be mentioned, which contains at least one ligand and one matrix, wherein the matrix can be a soluble polymer, of which examples are dextran or polyacrylamide, or a non-soluble polymer, of which examples are agarose or, in one embodiment, cross-linked agarose. Examples of ligands consist of one or more saccharides. These are bound via an aglycone, which is glycosidically bound to the reduced end of the saccharide, and the aglycone is covalently bound to the adsorbent.

Blood group A and B can be in the form of an A-trisaccharide determinant or a B-trisaccharide determinant, respectively. The blood group A determinant is glycosidically linked to additional carbohydrate structures containing for example GlcNAc or GalNAc. Thus, due to these additional saccharide structures, blood group A can be divided into different subtypes, i.e. subtype 1, 2, 3, 4, and additional subtypes. This is similar for blood group B and H. This means that in some cases patients develop anti-A and or anti-B antibodies which require longer structures than the A- or B trisaccharide determinant for stronger binding.

The blood group A-trisaccharide determinant is: GalNAcα1-3(Fucα1-2)Galβ1-.

Below are given two examples of each one of the blood group A subtypes 1, 2, 3 and 4, respectively:
GalNAcα1-3(Fucα1-2)Galβ1-3GlcNAcβ1-,
GalNAcα1-3(Fucα1-2)Galβ1-3GlcNAcβ1-3Galβ1-4Glcβ1-,
GalNAcα1-3(Fucα1-2)Galβ1-4GlcNAcβ1-,
GalNAcα1-3(Fucα1-2)Galβ1-4GlcNAcβ1-3Galβ1-4Glcβ1-,
GalNAcα1-3(Fucα1-2)Galβ1-3GalNAcβ1-,
GalNAcα1-3(Fucα1-2)Galβ1-3GalNAcβ1-3Galβ1-4Glcβ1-
GalNAcα1-3(Fucα1-2)Galβ1-3GalNAcα1-,
GalNAcα1-3(Fucα1-2)Galβ1-3GalNAcα1-3Galβ1-4Glcβ1-.

The blood group B-determinant, Galα1-3(Fucα1-2)Galβ- 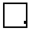 can be divided in blood group subtypes as described above for the A-ligand.
Galα1-3(Fucα1-2)Galβ1-3GlcNAcβ1-,
Galα1-3(Fucα1-2)Galβ1-3GlcNAcβ1-3Galβ1-4Glcβ1-,
Galα1-3(Fucα1-2)Galβ1-4GlcNAcβ1-,
Galα1-3(Fucα1-2)Galβ1-4GlcNAcβ1-3Galβ1-4Glcβ1-,
Galα1-3(Fucα1-2)Galβ1-3GalNAcβ1-,
Galα1-3(Fucα1-2)Galβ1-3GalNAcβ1-3Galβ1-4Glcβ1-,
Galα1-3(Fucα1-2)Galβ1-3GalNAcα1-,
Galα1-3(Fucα1-2)Galβ1-3GalNAcα1-3Galβ1-4Glcβ1-.

The blood group H determinant can also be divided into different subtypes, as described above.

One of the embodiments uses an aglycon, which separates the carbohydrate ligand from the matrix. A-, or B- or H-saccharide containing ligands can be obtained by linking the A-, B- or H structure glycosidically via the 1-position of the saccharide to an aglycon, i.e., according to the example above, in the α1- or β1- position (right end of each saccharide above). This is selected by the expert.

Optionally, as mentioned above, the adsorbent contains an A-trisaccharide, i.e. GalNAcα1-3(Fucα1-2)Galβ1-, together (optional) with one, two or more of the above structures, and/or together with (optional) a B-trisaccharide, i.e. Galα1-3(Fucα1-2)Galβ1-.

Example of an adsorbent according to the invention is a protein, or a peptide or carbohydrate bound directly to a matrix or via a monomeric, dimeric, oligomeric or polymeric aglycone to a matrix. In one embodiment the aglycon is monomeric, but in the case of for example a weaker binding, a dimeric or oligomeric aglycon (thus each aglycon, and thus ligand, contains two or more, respectively, thereto covalently bound carbohydrate molecules). There are numerous examples of dimeric or oligomeric molecules that can be used of which peptides is one category, to which peptides and carbohydrates can be bound covalently, and the expert in the field will select this.

The matrix can be soluble or non-soluble in buffer, blood, blood plasma or completely or partly purified blood plasma. Non-limiting examples of soluble matrix contains dextran, starch or starch derivatives, amylose, amylopectine, or polyacrylamide. In one embodiment the adsorbent is in the form of porous gel beads and contains cross linked agarose with thereto covalently bound ligand. The agarose based adsorbents form a suspension in buffer, blood or blood plasma.

As a non-limiting example of monomeric aglycone where the aglycone is glycosidically bound via -O- to the carbohydrate according to the example above, can be mentioned for example the monomeric aglycone which contains one or more of the structures -OPhNH-, -OEtPhNH- or -O(CH₂)ₙ-NH- where the NH group is bound directly to the matrix or is bound to the matrix via another chemical structure, which is one of the monomeric structures mentioned below. Alternatively the structure can be bound to di-, tri- or oligomeric structures as exemplified below, or to amino acid, peptide or protein.

As another example, a carbohydrate with the above type of glycosidically bound aglycone, is optionally bound to a matrix via any of the exemplified structures' amino acid group and another chemical structure, example of such a structure bound to the matrix is given below: -C(O)-(CH₂)ₙ-O-CH2-matrix or -(CH₂)ₙ-O-CH2-matrix, where n is a whole number, preferably n is 1, 2, 3, 4, 5, 6, or higher, in order to create further space between the carbohydrate ligand and the matrix.
Non-limiting examples of adsorbents constructed according to these example are thus carbohydrate-OEtPhNH-C(O)-(CH₂)ₙ-O-CH2-matrix, Peptide-NH-C(O)-(CH₂)ₙ-O-CH2-matrix,
carbohydrate-OEtPhNH-C(O)-(CH₂)ₙ-NH-CH2-matrix, peptide-NH-C(O)-(CH₂)n-NH-CH2-matrix.

Further non-limiting examples of ligands and of above mentioned carbohydrates and carbohydrate derivatives according to the present invention, are monosaccharide, disaccharide or oligosaccharide, GM1, other ganglioside structures, other sialylated structures, GalNAcbeta1-3Gal, GalNAcbeta1-4Ga, galili-antigen, carbohydrate which contain Galalfa1-4Gal structure, P-antigen, blood group A-, blood group B-, blood group H, or other carbohydrates found in glycoproteins, glycopeptides, or glycolipids.

Further non-limiting examples of ligands are mono-, di-, tri-, tetravalent and higher oligomers of peptides or of above exemplified saccharides, derivatives of the said saccharides for example containing O-, N-, or S-glycosides of these substances, where the aglycon comprises for example an aliphatic or aromatic part and for example the aglycon contains a terminal sulfhydryl-, hydroxyl-, amino- or carboxyl group for covalent binding to the matrix, e.g. the polymer particle or polymer bead as described above, or in which said carbohydrate derivatives contains at least one biotin, avidin or streptavidin molecule for noncovalent binding to an avidine, streptavidine or biotin-matrix.

The di-, tri-, tetra-, oligomeric aglycon can contain for example a molecule containing at least one or several -(CH₂)n-O- units, where n is 2, 3, 4 or a higher number, forming an oligomer by for example linkage to one or more other oligofunctional molecules. The di-, tri-, tetra-, oligomeric aglycon is constructed to be able to be linked O-, N- or S-glycosidically - or via another aglycon in between the carbohydrate and the-(CH₂)n-O- units - to one or several carbohydrates or carbohydrate derivatives, thereby forming a dimeric, trimeric, tetrameric, oligomeric carbohydrate derivative, which in turn is linked to matrix. The di-, tri-, tetra- or oligomeric ligand can be chosen by the expert to obtain a stronger binding to the product of the antibody or protein to be purified and/or minimize the size of the product for the specific application/use of the product. The exact structure of the aglycon is made by the expert in the field. The aglycon can also contain a peptide or protein.

Adsorbent with one or more different ligands can be used bound to matrix. The choice of the ligand and the concentration of the ligand on the matrix, the conditions of coupling ligand to matrix, is made by the expert for the specific application and do not limit the scope of the invention.

As another non-limiting example of ligand to be mentioned: A peptide, a protein, a glycopeptide or a glycoprotein. Non-limiting example of this is protein A, endopeptidase, antibody against a certain antigen, such as IgG or IgM or another antigen, one or more peptide able to bind antibody towards HLA antigen, antigen to antibody, such as acetylcholine receptor, avidin, or a peptide that can bind the desired component to be reduced in the blood or blood plasma or the completely or partly purified blood plasma. Carbohydrate structures which bind to toxins produced by bacteria, for example Shigella, Vibrio cholera, EHEC, other toxins (specific against other carbohydrate structures) produced by other disease inducing bacteria. For the purposes of the invention, these types of ligands can be bound directly to the matrix (binding occurs to matrix which has first been activated with a reactive group), or via a so called spacer, which separates the protein from the matrix as in the case above, this does not limit the scope of the invention.

Choice of ligand and binding method with conditions such as temperature, reaction time, buffer and concentration of ligand, are made by the specialist in the field and do no limit the scope of the invention. There are numerous methods available for chemical coupling of a ligand to a matrix and this does not limit the scope of the invention. As a non-limiting example of binding the ligand to matrix, can be mentioned the use of so called NHS activated matrix, (NHS, N-hydroxysuccinimide), where an amide bond is formed for example between a ligand amino (NH-) group and a carboxyl group on the matrix, as exemplified above. Non-bound ligand after coupling is removed from the adsorbent by washing the adsorbent with for example buffer PA and other buffer recommended for washing after coupling and as determined by the expert in the field.

As mentioned above, the coupling of ligand to matrix, is preferably performed in clean rooms under controlled and validated conditions, and sterilized by steam as
In a preferred embodiment, the matrix have a high porosity allowing entrance in to the pores of the beaded matrix also of larger components (such as for example IgG and IgM), desired to be reduced from e.g. whole blood or blood plasma by the treatment according to the invention. In a preferred embodiment according to the invention, the porosity of the beaded non-soluble matrix is maintained after coupling of the ligand to an extent to allow for binding of larger proteins and rapid entrance of smaller molecules and this can be achieved according to the invention by using e.g. the low molecular ligands described above.

Examples of preferred matrix is agarose based matrix, for example agarose or cross-linked agarose which normally is porous or macroporous, thus contains pores which allow entrance of proteins or antibodies into said pores where a large part of ligand is bound. As a non-limiting example matrix with 4 % dry weight agarose or 4 % dry weight cross linked agarose is used. In another non-limiting example, cross-linked agarose is used with a lower agarose dry weight content, 2 or 3 % dry weight, or a value in between, to allow faster access to the pores inside the matrix for binding of larger plasma components such as IgG and/or IgM antibodies.

Non-limiting examples of specific antibodies are anti-A, anti-B, anti-GM1, anti-galalfal -4Galbeta1-4Glc antibodies, other antibodies specific for other ganglioside, glycolipid and glycoprotein carbohydrate structures. An example of commercially available agarose matrix, which can be used according to the invention is Sepharose 2B or CL 2B where CL is a cross linked variant of agarose, but agarose of higher dried content can be used, such as the commercially available Sepharose 4B or CL4B, CL 6B or Sepharose 4FF. Also other types of agarose can be used and the choice of agarose, pore size, sixe of agarose beads, amount of agarose based product during the use of the invention, is made by the specialist and does not limit the scope of the invention. In one preferred embodiment a more highly cross-linked agarose than for example conventional cross-linked agarose is used, the commercially available Sepharose 4 FF is an example of such preferred matrix. The higher cross-linking gives a higher resistance towards disintegration in the plasma filter.

In the example of a carbohydrate ligand, the covalent binding to the matrix is preferably stable during use according to the invention and this type of binding has been exemplified above. Non-limiting examples are amide (ligand-NH-CO-, ligand-CO-NH-), N-C or O-C binding. The first of these three exemplified bindings, the amide binding, is formed for example through the reaction of an activated carboxyl group (activated with carbodiimide and / or N-hydroxysuccinimid) on a carboxyl group containing matrix (or ligand) with an amino group containing ligand (or matrix respectively). The reverse can also be carried out, i.e. activating a carboxyl group containing ligand (in the same way) and coupling to an amino group containing matrix. Coupling between ligand and matrix can also be performed with the help of so called cross binders, which are reactive against e.g. amino groups. This does not limit the scope of the invention and the choice of method and conditions is made by the specialist.

As a non-limiting example, the invention can be used for the reduction of toxins, immunoglobulins, specific antibodies such as GM1 antibodies, antibodies against other gangliosides, antibodies, peptides, HLA-antigen, acetylcholine receptor, anti-A and/or anti-B and/or anti-H antibodies, antibodies against for example acetylcholine receptor and antibodies directed against other structures, toxins and towards antibodies in certain autoimmune conditions.

The invention can be used for example in connection with transplantation, or in connection with blood transfusion or plasma transfusion for preparation of human plasma or freeze-dried human plasma with reduced anti-A and/or anti-B, and/or anti-H antibody content, in the preparation of intravenous immunoglobulin (e.g. with reduced anti-A and or anti-B content) or for preparation of other plasma components.

When a polymer product in the form of gel beads is used, the size of the gel beads will be chosen by the specialist and does not limit the scope of the invention. For example, the plasma component will bind smaller gel beads faster. For example, for the treatment of plasma in the range of 20 - 200 micrometer or a less wide distribution in size within this size Beaded cross-linked agarose, with an average bead size in the range 70-100 is used. The type of cross-linked agarose is selected by the expert. In one preferred embodiment, a higher degree of cross-linking of the matrix (as in commercially available Sepharose 4FF) can be chosen by the expert instead of an agarose with a lower degree of cross-linking (for example commercially available Sepharose CL2B, CL4B) to improve for example the flow properties and decrease the risk for disintegration of the adsorbent suspension in the plasma.

The amount of adsorbent per volume blood or blood plasma is adjusted by the specialists just as the flow through the filter and its pore size and design.

As a non-limiting example, 0.1 ml, 1 ml, 2 ml, 30 ml or 60 ml adsorbent can be used or any value in between these values, during the treatment of between for example 100ml to 10 L. Larger amounts or amounts in between these, of the adsorbent can be used depending on the application. The contact time, temperature and other parameters can be chosen by the specialist and do not limit the scope of the invention.

Generally, the amount of adsorbent will be adjusted to the blood or blood plasma volumes set to be treated.

## Claims

1. A method for the reduction or elimination of one or more components from a blood product from a container or a blood bag, wherein it comprises continuous addition of the blood product to a filter containing an inlet for the blood product, an outlet for the blood product in which said one or more components have been reduced or eliminated, hollow fiber membranes having pores through which said one or more components and blood plasma may pass outwardly, a space between the hollow fiber membranes, a blood plasma outlet, a blood plasma inlet, and a loop connected between said blood plasma outlet and said blood plasma inlet, wherein said space between the hollow fibers in the filter and the loop contains an adsorbent which specifically binds said one or more components, wherein the pores of the hollow fibers do not allow passage of the adsorbent into the hollow fiber membranes, wherein the blood plasma and the adsorbent is continuously recirculated via the loop back to the filter and to the space between the hollow fiber membranes, and wherein when the concentration of said one or more components in the blood product which exits the filter via the blood product outlet reaches a pre-determined value, the addition of blood product is stopped and **characterized in that** said adsorbent comprises at least one matrix having at least one covalently bound ligand thereto, wherein said matrix is beaded cross-linked agarose with an average bead size in the range 70-100 micrometer.

2. The method according to claim 1, wherein the blood product is whole blood, blood plasma, or completely or partially purified blood plasma, or an intravenous immunoglobulin preparation.

3. The method according to claim 1 or 2, wherein the adsorbent having said one or more components bound thereto is separated from the filter, and said one or more components then are eluted from the adsorbent.

4. The method according to claim3, wherein the ligand is one or more saccharides, bound to the matrix via an aglycon, or is a peptide, an antibody, or a protein.

5. The method according to claim 4, wherein the ligand comprises one or more blood group A and/or blood group B saccharides.

6. The method according to claim 1, wherein the adsorbent is supplied in the loop before connecting the loop to the filter.

7. The method according to claim 1, wherein the flow rate of the loop is higher than the flow rate of the blood product or liquid entering the filter.

8. The method according to claim 7, wherein the flow rate of the blood product or liquid entering the filter is about 50 mL to about 150 mL per minute and the flow rate in the loop is about 100 mL to about 400 mL per minute.

9. The method according to claim 1, wherein the filter has a pore size of about 0.1 µm to about 0.3 µm.

10. The method according to claim 1, wherein the matrix is a soluble polymer.

## Patentansprüche

1. Verfahren zur Verringerung oder Eliminierung einer oder mehrerer Bestandteile aus einem Blutprodukt aus einem Behälter oder einem Blutbeutel, wobei es die kontinuierliche Zugabe des Blutprodukts in einen Filter umfasst, der einen Einlass für das Blutprodukt, einen Auslass für das Blutprodukt, in dem der eine oder die mehreren Bestandteile verringert oder eliminiert wurden, Hohlfasermembranen mit Poren, durch die der eine oder die mehreren Bestandteile und das Blutplasma nach draußen hindurchgehen, einen Zwischenraum zwischen den Hohlfasermembranen, einen Blutplasmaauslass, einen Blutplasmaeinlass und eine Schleife, die zwischen dem Blutplasmaauslass und dem Blutplasmaeinlass verbunden ist, enthält, wobei der Zwischenraum zwischen den Hohlfasern in dem Filter und der Schleife ein Adsorbens enthält, das den einen oder die mehreren Bestandteile spezifisch bindet, wobei die Poren der Hohlfasern den Durchgang des Adsorbens in die Hohlfasermembranen nicht erlauben, wobei das Blutplasma und das Adsorbens kontinuierlich über die Schleife zu dem Filter und zu dem Zwischenraum zwischen den Hohlfasermembranen zurückgeführt wird, und wobei, wenn die Konzentration des einen oder der mehreren Bestandteile in dem Blutprodukt, das den Filter über den Blutproduktauslass verlässt, einen vorbestimmten Wert erreicht, die Zugabe des Blutprodukts anhält und **dadurch gekennzeichnet ist, dass** das Adsorbens mindestens eine Matrix mit mindestens einem kovalent daran gebundenen Liganden umfasst, wobei die Matrix perlenförmig vernetzte Agarose mit einer mittleren Perlengröße im Bereich von 70-100 Mikrometern ist.

2. Verfahren nach Anspruch 1, wobei das Blutprodukt Vollblut, Blutplasma oder vollständig oder teilweise gereinigtes Blutplasma oder ein intravenöses Immunglobulinpräparat ist.

3. Verfahren nach Anspruch 1 oder 2, wobei das Adsorbens, das den einen oder die mehreren Bestandteile daran gebunden aufweist, aus dem Filter separiert wird, und der eine oder die mehreren Bestandteile dann aus dem Adsorbens eluiert werden.

4. Verfahren nach Anspruch 3, wobei der Ligand ein oder mehrere Saccharide, die über ein Aglykon an die Matrix gebunden sind, oder ein Peptid, ein Antikörper oder ein Protein ist.

5. Verfahren nach Anspruch 4, wobei der Ligand eine oder mehrere Saccharide der Blutgruppe A und/oder Blutgruppe B umfasst.

6. Verfahren nach Anspruch 1, wobei das Adsorbens der Schleife zugeführt wird, bevor die Schleife mit dem Filter verbunden wird.

7. Verfahren nach Anspruch 1, wobei die Strömungsrate der Schleife höher ist als die Strömungsrate des Blutprodukts oder der Flüssigkeit, die in den Filter eintritt.

8. Verfahren nach Anspruch 7, wobei die Strömungsrate des Blutprodukts oder der in den Filter eintretenden Flüssigkeit etwa 50 mL bis etwa 150 mL pro Minute beträgt und die Strömungsrate in die Schleife etwa 100 mL bis etwa 400 mL pro Minute beträgt.

9. Verfahren nach Anspruch 1, wobei der Filter eine Porengröße von etwa 0,1 µm bis etwa 0,3 µm aufweist.

10. Verfahren nach Anspruch 1, wobei die Matrix ein lösliches Polymer ist.

## Revendications

1. Procédé de réduction ou d'élimination d'un ou plusieurs composants à partir d'un produit sanguin provenant d'un récipient ou d'une poche de sang, où celui-ci comprend l'ajout continu du produit sanguin à un filtre contenant une entrée pour le produit sanguin, une sortie pour le produit sanguin dans lequel lesdits un ou plusieurs composants ont été réduits ou éliminés, des membranes de fibres creuses ayant des pores à travers lesquels lesdits un ou plusieurs composants et le plasma sanguin peuvent passer vers l'extérieur, un espace entre les membranes de fibres creuses, une sortie de plasma sanguin, une entrée de plasma sanguin, et une boucle reliée entre ladite sortie de plasma sanguin et ladite entrée de plasma sanguin, dans lequel ledit espace entre les fibres creuses dans le filtre et la boucle contient un adsorbant qui se lie spécifiquement auxdits un ou plusieurs composants, dans lequel les pores des fibres creuses ne permettent pas le passage de l'adsorbant dans les membranes de fibres creuses, dans lequel le plasma sanguin et l'adsorbant est recirculé en continu par l'intermédiaire de la boucle de retour vers le filtre et vers l'espace entre les membranes de fibres creuses, et dans lequel, lorsque la concentration desdits un ou plusieurs composants dans le produit sanguin qui sort du filtre par la sortie de produit sanguin atteint une valeur prédéterminée, l'ajout de produit sanguin est arrêté et **caractérisé en ce que** ledit adsorbant comprend au moins une matrice ayant au moins un ligand lié de façon covalente à celle-ci, dans lequel ladite matrice est constituée de billes d'agarose réticulée avec une taille de bille moyenne dans la plage de 70 à 100 micromètres.

2. Procédé selon la revendication 1, dans lequel le produit sanguin est du sang total, du plasma sanguin, ou du plasma sanguin complètement ou partiellement purifié, ou une préparation d'immunoglobuline intraveineuse.

3. Procédé selon la revendication 1 ou 2, dans lequel l'adsorbant ayant lesdits un ou plusieurs composants liés à celui-ci est séparé du filtre, et lesdits un ou plusieurs composants sont ensuite élués depuis l'adsorbant.

4. Procédé selon la revendication 3, dans lequel le ligand est un ou plusieurs saccharides, liés à la matrice par l'intermédiaire d'un aglycone, ou est un peptide, un anticorps ou une protéine.

5. Procédé selon la revendication 4, dans lequel le ligand comprend un ou plusieurs saccharides de groupe sanguin A et/ou de groupe sanguin B.

6. Procédé selon la revendication 1, dans lequel l'adsorbant est introduit dans la boucle avant de relier la boucle au filtre.

7. Procédé selon la revendication 1, dans lequel le débit de la boucle est supérieur au débit du produit sanguin ou du liquide entrant dans le filtre.

8. Procédé selon la revendication 7, dans lequel le débit du produit sanguin ou du liquide entrant dans le filtre est d'environ 50 ml à environ 150 ml par minute et le débit dans la boucle est d'environ 100 ml à environ 400 ml par minute.

9. Procédé selon la revendication 1, dans lequel le filtre a une taille de pore d'environ 0,1 µm à environ 0,3 µm.

10. Procédé selon la revendication 1, dans lequel la matrice est un polymère soluble.
